# EUROPEAN PATENT APPLICATION

(11) **EP 3 900 755 A1**
(43) Date of publication of application: **27.10.2021**
(21) Application number: 21169678.6
(22) Date of filing: 21.04.2021
(51) Int. Cl.: A61M 1/34

(54) **INTEGRATED PLASMAPHERESIS, HAEMOFILTRATION, RECIRCULATION AND PLASMA CONCENTRATION PLATFORM FOR PATHOGEN DIAGNOSIS AND OTHER BIOMEDICAL APPLICATIONS**

(30) Priority: 22.04.2020 IT 202000008677
(71) Applicant: Ceinge Biotecnologie Avanzate SCarl, 80131 NAPOLI (IT)
(72) Inventor: TOMAIUOLO, Giovanna, 80131 NAPOLI (IT); PREZIOSI, Valentina, 80131 NAPOLI (IT); GUIDO, Stefano, 80131 NAPOLI (IT)
(74) Representative: Bianchetti & Minoja SRL

(57) **Abstract**

Disclosed is a system for improving the efficiency of systems designed for filtration of blood or other biological fluids, whether or not they include cell suspensions, comprising a water separation and recovery unit, which allow:
- the implementation of some organ functions using extracorporeal devices;
- purification of blood or fluid from toxins due to a disorder;
- plasma concentration (including in miniaturised devices) for diagnostic purposes.

## Description

The invention relates to a system for filtration of blood or other biological fluids and a device for the implementation thereof.

### PRIOR ART

Blood analysis and treatment devices have a wide range of biomedical applications, comprising artificial organs, diagnosis of infectious agents (such as viruses and bacteria), production of blood derivatives and purification of blood from toxins and inflammatory mediators such as cytokines. A common aspect of said devices is extracorporeal blood treatment, which may be continuous, with reinfusion of treated blood into the patient's circulatory system, or discontinuous, involving taking a sample for subsequent offline analysis, as in the case of diagnosis of infectious agents. The various blood treatment processes can be classified in terms of operations common to several devices: 1) blood pumping, 2) separation of plasma from corpuscular components (plasmapheresis), 3) haemofiltration, 4) material exchange (e.g. by adsorption), 5) heat exchange, 6) device monitoring and control systems. Each of these operations presents various difficulties, not all of which have been solved, especially in the therapeutic field, mainly due to the fact that blood is manipulated in an extracorporeal environment.

For example, the flow conditions imposed during extracorporeal circulation, in particular the possible onset of turbulent motion, can damage the corpuscular components (haemolysis) and activate an inflammatory response, with possible thrombogenic results. The latter can also be triggered by biochemical interactions with the materials used to make the inner surfaces of the channels through which the blood is conveyed. Further problems are due to the low transport efficiency of material in membrane processes; said transport is mainly based on diffusion mechanisms, and therefore requires large volumes of ultrapure water, with the addition of ingredients suitable to maintain the osmotic balance and pH of the plasma, namely the medium to which the blood toxins are transferred.

A critical aspect of plasmapheresis and haemofiltration in particular relates to fouling of the membranes used in said operations. (Guo, W. et al., A mini-review on membrane fouling. Bioresource technology 2012, 122, 27-34). Fouling is generally associated with the concentration polarisation effect, whereby a fluid layer with a high concentration of ingredients such as electrolytes and proteins forms near the membrane surface, and can give rise to precipitates and deposits, leading to clogging of the membrane pores and a rapid reduction in permeate flow over time, which can fall to values well below the theoretical capacity of the membrane. In the case of biofouling, the material consists of micro-organisms and the by-products thereof, such as extracellular polysaccharides and metabolites (Magin, C. M. et al., Non-toxic antifouling strategies. Materials today 2010, 13 (4), 36-44). Fouling, which therefore limits the performance and duration of membrane systems in numerous applications, is caused by complex chemical and physical interactions between the constituents of the material deposited, and between said constituents and the membrane surface. This aspect represents a considerable problem, because of the high cost of the membranes and the inevitable subsequent stage of replacement thereof, involving a temporary stoppage of the process.

Chemical modification of the membrane surface is considered to be a technology of considerable interest, designed to improve the performance and duration of the membrane; said process, called "anti-fouling", involves preventing undesirable attack by organisms in the surface area (Shannon, M. A et al., Science and technology for water purification in the coming decades. In Nanoscience and technology: a collection of reviews from nature Journals, World Scientific: 2010; pp 337-346). Increased hydrophilicity improves the anti-fouling properties of membranes, because the majority of natural biopolymers exhibit hydrophobic properties. Theoretically, hydrophilic surfaces create a buffer layer that prevents foulers from adhering, and this gives rise to a stable, high flow through the membrane. The possibility of incorporating membranes in flow channels to compartmentalise them is important to control the flow between compartments, to exclude entire components, or to introduce materials selectively via filtration, by excluding antagonist substances. Integrated devices of this kind can offer major benefits in both the pharmaceutical and the biomedical field.

The devices currently available in clinical practice are only able to perform some blood purification functions, such as removal of cytokines and inflammatory agents for treatment of infections, or release of some substances, such as the oxygen used for extracorporeal oxygenators, and insulin, as in the case of the artificial pancreas. More complex biochemical functions, such as metabolic and immunological functions, are not currently available, but a great deal of research is being conducted in said field. One of the most widespread lines of research is based on introducing cultures of living cells (liver, cardiac, etc.) into extracorporeal devices, thus implementing a bioartificial system with the aim of obtaining a broader spectrum of biochemical function. However, it should be emphasised that the difficulties mentioned to date relating to the artificial organs used in clinical practice also have an adverse impact on the design of bioartificial devices, and therefore constitute a limiting factor for the technological development of more advanced systems designed to replace vital organ functions.

In addition to therapeutic applications, treatment of blood samples is also an essential aspect of the operation of many diagnostic devices. The progress of analysis techniques has allowed further exploitation of the diagnostic potential of blood in applications such as point-of-care analysis, designed to allow rapid detection of physiological parameters from a sample taken at the place of treatment for emergency reasons, in remote patient management, and for diagnosis in premises with limited health infrastructures (as in the developing countries). These aspects are particularly interesting in the case of epidemics, which often require large-scale diagnostic screening, which is facilitated if the analyses can be conducted in point-of-care mode or at premises lacking specialist facilities, such as airports and railway stations. Another recent example is the characterisation of a blood sample as a "liquid biopsy", in order to obtain useful information for the prognosis and treatment of some disorders. Some of the difficulties cited for artificial organs also arise in diagnostic applications. One example is separation of plasma from the corpuscular components, which allows more effective analysis, but is not easy to conduct on a limited blood volume originating from a sample taken in discontinuous mode.

The filters most widely used for extracorporeal devices consist of bundles of hollow fibres with an inner diameter of around 200 microns and walls made of a polymer such as polyethersulphone, which act as a porous membrane. The fibre bundles are housed in plastic cylinders with a diameter of about 5 cm and a length of about 30 cm. The blood originating from the patient flows through the hollow fibres, while the blood purification fluid flows on the outside. One of the drawbacks of this configuration is that the membranes tend to clog with time due to the deposit of substances such as proteins and salts around the pores.

Analysis of the literature shows that research into new technologies designed to overcome the limitations of the current technology is based on both artificial and biological approaches. In both cases, research activity has mainly focused on developing more efficient membranes and smaller devices. In some studies, the membranes were coated with cells to improve blood filtration and also reproduce other functions. These biological approaches, which aim to implement an implantable device as a long-term objective, still suffer from numerous unsolved problems, including limited cell survival in an artificial device, and the need to obtain a large number of cells.

US 2018/0141003 describes both nanoporous membranes and living cells cultured on said membranes. The principle used is that of ultrafiltration: applying a certain pressure to the blood originating from the patient causes the toxins to pass through the nanopores in the membrane, compounds with a higher molecular weight being retained, while the presence of cells should provide further functions in addition to filtration. The membrane is made of silicon or other materials using microfabrication techniques to obtain pores of nanometric dimensions with different geometries (in particular, rectangular channels to increase the efficiency of filtration). The possibility of coating the membrane (e.g. with PEG) to reduce clogging, applying an electrical field to promote separation of charged species and integrating the device with pumps, actuators and monitoring and control systems, has also been considered. Uses in the fields of diagnostics, bioreactors for cell cultures and bioartificial organs are indicated, with specific reference to the manufacture of portable or implantable miniaturised devices.

US 2011/ 0040228 describes a system for removal of toxins from a patient's blood, with reinfusion of purified blood. The system involves partial recovery of the aqueous component of plasma, which is added to the purified blood together with a supplementary fluid, mainly consisting of a balanced solution of salts and albumin, to offset plasma losses during blood purification. The haemofiltration unit on which the system is based involves the use of membranes with fairly large pores to allow the passage of high-molecular-weight ingredients. The main drawback of this approach, as regards the purposes of the present invention, is that the corpuscular components are separated by a plasma stream containing both low- and high-molecular-weight substances, whereas separation of molecular weights is essential in various applications.

More recently, a method for recycling at least part of the water lost through spent process fluids was described in US 10,293,094. Water is recycled using membranes comprising vesicles whose bilayer incorporates acquaporin channels which are highly selective for water. The driving force for the passage of water through the membranes is osmosis (forward osmosis or FO) induced by the presence of a salt gradient. The possible clinical application of FO has been suggested as a basis for slower, more continuous treatments than the conventional ones (Talaat, K. M., Dialysis fluid regeneration by forward osmosis: a feasible option for ambulatory dialysis systems. Saudi Journal of Kidney Diseases and Transplantation 2010, 21 (4), 748), with the above-mentioned advantages of more efficient removal of phosphates and protein-bound uraemic toxins, and better control of cardiovascular volume and stability. Talaat, imagining the use of NaCl as salt for the draw solution in FO, estimated water recovery below 50%, and postulated the introduction of membranes with small priming volumes, low thrombogenicity, high permeability for the larger uraemic toxins, and better biocompatibility. Of the possible membranes, asymmetrical cellulose membranes which exhibit a high rate of salt rejection have been proposed (US 7,445,712), although they are not equally effective for urea, as have polymer membranes incorporating acquaporin channels (Kumar, M. et al., Highly permeable polymeric membranes based on the incorporation of the functional water channel protein Aquaporin Z. Proceedings of the National Academy of Sciences 2007, 104 (52), 20719-20724), which were later patented as a coating for hollow fibres (WO 2014/10882). However, recovery of water by filtration still remains an ambitious objective to achieve with a biomedical device because of various technological difficulties, such as the above-mentioned fouling, and the need to integrate the recovery unit into the device. The latter aspect requires a novel engineering design, not yet available in the literature, designed to control the flow rates and pressures in the inflow and outflow lines to and from the water recovery unit to take account of the effects of fouling and, if necessary, reuse the recovered water in the other units of the device. For example, US 2012/0283628 describes a dialysis system not including a water recovery unit for plasma concentration before reinfusion of the purified blood.

### DESCRIPTION OF THE INVENTION

It has now been found that the operational difficulties involved in extracorporeal devices, whether therapeutic or diagnostic, can be overcome by integrating operations common to a plurality of devices in an innovative scheme that allows greater efficiency of treatment and therefore requires smaller items of equipment and more limited use of auxiliary fluids. Miniaturisation of extracorporeal blood treatment devices is a major technological issue with applications of considerable impact, such as point-of-care diagnosis and therapeutic blood purification processes.

In particular, it has been found that the efficiency of filtration systems for blood or other biological fluids, whether or not they include cell suspensions, can be improved by introducing water separation and recovery units which allow:
- the implementation of some organ functions using extracorporeal devices;
- purification of blood or fluid from toxins due to a disorder;
- concentration of plasma (including in miniaturised devices) for diagnostic purposes.

The water recovery units usable according to the invention preferably act by direct or reverse osmosis.

The invention, in a first aspect thereof, provides a miniaturised filtration system for blood or other biological fluids, whether or not they include cell suspensions, designed to treat fluid flow rates starting from microlitres/minute in a version for portable or domestic use, which comprises:
a) means for separating plasma or suspending fluid from the corpuscular components;
b) a unit for filtration of plasma or an aqueous solution able to provide a fraction enriched in proteins, DNA, RNA, pathogens, inflammation mediators or high-molecular-weight substances and a fraction enriched in low-molecular-weight solutes;
c) a water recovery unit for further concentration of the fraction enriched in proteins, DNA, RNA, pathogens, inflammation mediators or high-molecular-weight substances, and the integration thereof with the other units of the system via recycle streams and auxiliary streams to dilute the corpuscular components separated in a) and allow the operation of the filtration unit in step b);
d) means for detection of the target analytes in the fraction enriched in proteins, DNA, RNA, pathogens or high-molecular-weight substances from step c), and/or means of separating undesirable ingredients present in said fraction;
e) means for recycle of the fraction analysed or purified in step d), after mixing with the fraction enriched in low-molecular-weight solutes from step b), and with the corpuscular components from separation step a).

The system according to the invention, in one embodiment thereof, also comprises the addition of an auxiliary solution to water recovery unit c) and recycle of water from unit c) to units a) and b).

Stage d) can comprise taking a sample of a portion of the enriched fraction for analysis and recycle of the remaining portion, after mixing with the fraction enriched in low-molecular-weight solutes obtained in step b), and with the corpuscular components obtained in separation step a), for the reconstitution of whole blood.

The means of separation described in step d) comprise an albumin-bound toxin adsorption unit and means for recycle of the purified fraction, after mixing with the fraction enriched in low-molecular-weight solutes obtained in step b) and with the corpuscular components obtained in separation step a), for the reconstitution of whole blood.

The water filtration and recovery units preferably comprise membranes made of cellulose, polysulphone or polyethersulphone, functionalised with catecholamines, such as dopamine.

In a preferred embodiment, the membrane is inserted in a two-compartment unit, in one of which fresh or protein-enriched plasma flows, while a recycle stream consisting of plasma diluted in protein flows in the other.

### DETAILED DESCRIPTION OF THE INVENTION

The filtration systems according to the invention will now be described in greater detail, with reference to the annexed figures, wherein:
Figure 1 illustrates a blood treatment scheme for diagnostic applications.
Figure 2 illustrates the operational scheme of an extracorporeal device designed to perform some hepatic blood purification functions.
Figure 3A illustrates the scheme of a unit with two compartments separated by a membrane.
Figure 3B illustrates the scheme for manufacturing the device shown in Figure 3A by 3D printing.

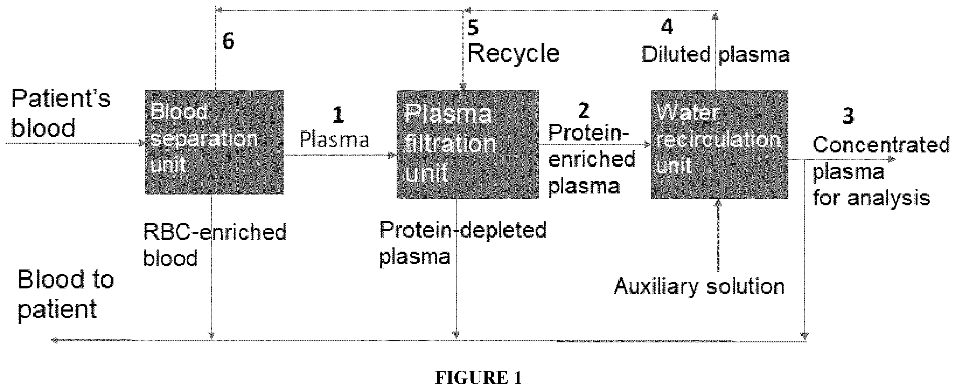

Figure 1 relates to a system that allows continuous blood treatment involving constant monitoring of proteins or other high-molecular-weight substances (circulating DNA, exosomes, cell membrane fragments, etc.), the blood being returned to the patient, except for a small portion of concentrated plasma retained for use in further analysis stages. A similar scheme, without the return of blood to the patient, can be used to diagnose viral or bacterial infectious agents and antibodies.

The system features an input line from the patient's circulatory system and a return line downstream of the blood manipulation unit (some components, such as pumps, valves, blood pressure and temperature monitors, and heat controls, have been omitted for the sake of simplicity).

The blood firstly undergoes an operation involving separation of plasma from the corpuscular components (red blood cells, white blood cells and platelets) by means of membrane processes or other processes (inertial, electrophoretic, or combined techniques) in the blood separation unit. The plasma thus obtained (stream 1) passes into the plasma filtration unit wherein it is enriched in proteins and other high-molecular-weight substances, separating them from the low-molecular-weight solutes. The separation unit comprises a membrane that separates two flow cells: fresh plasma originating from the blood separation unit flows into one, while a stream of diluted plasma originating from the subsequent water recovery operation, described below, flows into the other. The membrane that separates the flow cells consists of a blood-compatible material (such as polysulphone), and has a porosity such that it is essentially impermeable to high-molecular-weight substances. The passage of the low-molecular-weight solutes from fresh to diluted plasma takes place via diffusion and convection mechanisms through the membrane pores. The plasma thus enriched in high-molecular-weight substances (stream 2) flows into a water recycle unit, also based on a filtration membrane (for example, via a reverse osmosis process). In said unit the plasma is further concentrated in high-molecular-weight substances and conveyed (stream 3) to a subsequent analysis section (not shown in the figure) where the desired species are monitored. An auxiliary solution is added to the water recovered to maintain the pH and osmolarity in the physiological range, and said water is conveyed partly to the plasma filtration unit and partly to the blood separation unit. The outflow streams from said units finally reach the return line to the patient, wherein they are mixed with the plasma fraction enriched in red blood cells obtained in the filtration unit. As the analysis techniques require limited sample volumes, it can be estimated that the blood flow rates to be treated according to the scheme in Figure 1 are around 0.1 - 1 ml/minute.

By establishing a unitary base for the blood inflow rate to which all the flow rates refer, an outflow rate of concentrated plasma for the subsequent analyses can be estimated at 0.1 (stream 3 in Figure 1), and an auxiliary solution flow rate of 0.1. Assuming total separation of the corpuscular components in the blood separation unit (and a PCV of 45%), stream 1 would be 0.55. A flow rate of 0.4 can be estimated for outflow stream 2 from the plasma filtration unit. The flow rate of the outflow stream from the water recycle unit would be 0.1 on the basis of an equal distribution thereof in stream 3 and in the return stream to the patient. With these values, diluted plasma outflow stream 4 from the water recycle unit amounts to 0.3. The material balance is closed by assigning to streams 5 and 6 the flow rate values of 0.2 and 0.1 respectively.

Even for such small volumes, a return of blood to the patient is required for the purpose of continuous monitoring. The alternative procedure of discontinuous sample-drawing gives rise to a blood loss which can become significant over a fairly long period. The scheme also provides for the return to the patient of part of the plasma enriched in high-molecular-weight substances to prevent excessive loss of protein and other blood components.

The same sequence of operations as indicated in Figure 1 (except for the return to the patient) can also be applied to a discontinuous analysis procedure, involving taking a single blood sample to measure the desired parameters. The treatment stages illustrated in Figure 1 are useful to remove the corpuscular components and enrich the sample with high-molecular-weight substances, two aspects of considerable impact on the sensitivity and specificity of the measurement, for example for detection of immunoglobulins in patients suffering from an infectious disease.

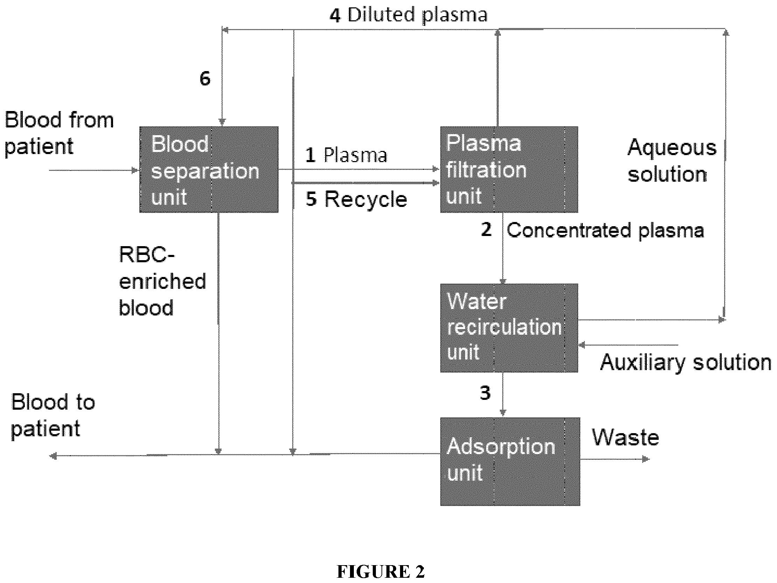

Figure 2 illustrates the scheme of a blood purification system for an artificial liver.

The scheme of the device has various aspects in common with the scheme shown in Figure 1.

The blood originating from the patient is conveyed to two plasma separation and filtration units as in Figure 1. The plasma filtration unit consists of two compartments separated by a membrane impermeable to proteins, and to high-molecular-weight substances in general. Fresh plasma (stream 1) flows in one compartment, while a recycle stream consisting of plasma diluted in proteins flows through the other. The fresh plasma is enriched in proteins by filtration through the membrane (under the action of diffusion and convection mechanisms), and then conveyed to a water recycle unit to further increase the protein concentration. The water recycle unit operates by means of a membrane process (optionally reinforced with water extraction using a direct- or reverse-osmosis mechanism), and the resulting aqueous solution is added to the outflow stream of protein-diluted plasma from the plasma filtration unit to obtain stream 4. The outflow of concentrated plasma from the water recycle unit (stream 3) is conveyed to an adsorption unit wherein the albumin-bound toxins are removed. The purified plasma, together with a fraction of the protein-diluted plasma stream, is mixed with the enriched plasma of the corpuscular components originating from the blood separation unit, and reinfused into the patient. Part of the protein-diluted plasma stream (stream 6) is recirculated in the blood separation unit to improve the efficiency of the process.

Once again, by establishing a unitary base for the blood inflow rate to which all the flow rates refer, a waste outflow rate (containing the albumin-bound toxins) of 0.05, and an auxiliary solution flow rate of 0.05, can be estimated. Assuming total separation of the corpuscular components in the blood separation unit (and a PCV of 45%), stream 1 would be 0.55. A flow rate of 0.4 can be estimated for outflow stream 2 from the plasma filtration unit. The flow rate of outflow stream 3 from the water recycle unit would be 0.25, while that of the recirculated aqueous stream would be 0.2. If the value of 0.35 is assigned to the flow rate of the other outflow stream from the plasma filtration unit, diluted plasma stream 4 amounts to 0.55. The material balance is closed by assigning to streams 5 and 6 the flow rate values of 0.2 and 0.1 respectively.

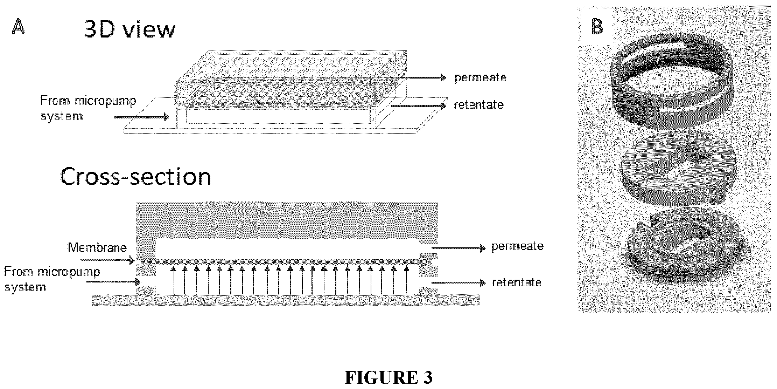

Figure 3a schematically illustrates a membrane unit which can be used for both blood separation and plasma filtration. As illustrated in the cross-section, the unit consists of two compartments separated by a membrane. The upper compartment in the figure is used for the permeate (the stream that passes through the membrane), and the lower compartment for the retentate (the outflow stream that exits the device without passing through the membrane). The inflow stream is conveyed to the unit via a feed device (consisting, for example, of micropumps for small flow rates). The same scheme also applies to the water recycle unit.

Figure 3b illustrates another embodiment of the same scheme, obtained by 3D printing. Once again, the unit consists of two compartments separated by a membrane (not shown in the figure). The inflow and outflow streams pass through the holes shown in the figure. The upper part of the scheme illustrates the lid which is screwed onto the two lower compartments to close the unit. The circular groove in the lower compartment houses an O-ring.

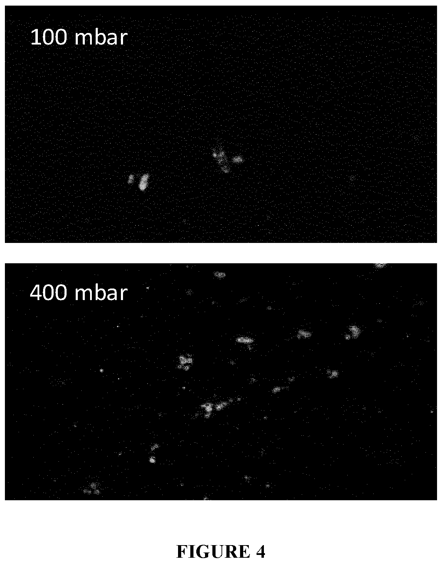

To drastically reduce the problem of fouling, the membranes undergo surface functionalisation treatment, which is particularly suitable for devices operating under flow, as schematically illustrated in Figure 3. Synthetic membranes consisting of polyethersulphone (PES) with a pore diameter of 0.2 µm are preferred. Confocal microscopy observation of the membranes subjected to the flow of PBS solutions (phosphate-buffered saline, pH 7.4) containing 10% by weight of bovine serum albumin (BSA) demonstrates that the presence of albumin aggregates (FITC-labelled) increases in proportion to the pressure set (Figure 4).

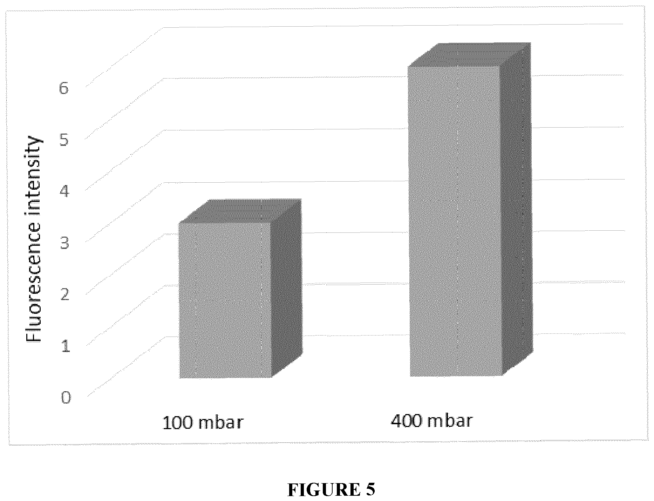

This effect was quantified by evaluating the fluorescence intensity (Figure 5).

The behaviour of PES membranes integrated into flow systems and functionalised with catecholamines was then analysed. Recently, a great deal of research has investigated possible coatings for use on the membranes, but none of them has ever analysed the behaviour of functionalised membranes *in situ* in flow systems. PES, like the majority of polymer membranes, possesses low hydrophilicity, thus limiting its performance and applications. For example, when PES membranes are used in water treatment processes, the water flow inevitably decreases because of fouling. Consequently, to make PES membranes usable in many fields, numerous research projects have aimed to modify the surfaces of said membranes, for example with surface treatments.

The coating is prepared with the use of buffer solutions, required to stabilise the pH at a desired value. For the dopamine-based solution (DA), TRIS-HCl at pH 8.8 is used as buffer agent.

The compartments (lower and upper) of the device are filled (with the aid of a syringe) so as to completely wet the membrane inserted in it with the dopamine solution, which is left to act for about 1 hour, followed by washing with ethanol for about 10 minutes and finally with distilled water for about 30 minutes. The washing process is required to eliminate the excess polydopamine.

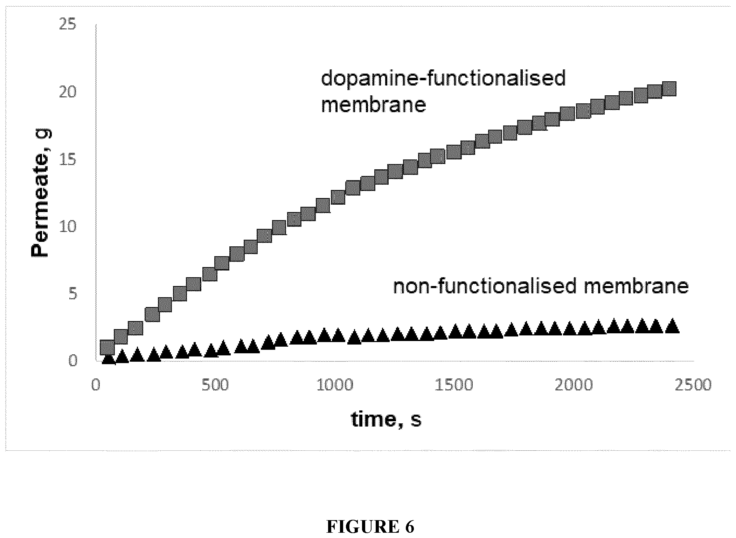

Figure 6 shows the trend over time of permeate passing through a PES membrane with 0.2 µm pores for a PBS (phosphate buffered saline, pH 7.4) solution containing 10% by weight of bovine serum albumin (BSA). The difference between the permeate without surface functionalisation treatment with dopamine (triangles) and with treatment (squares) is shown. The pressure is set to 20 mbars.

Microscopic observation demonstrates the presence of a deposit of aggregates that clog the pores of the membrane after the passage of the albumin solution for about 1 h at 20 mbars. After surface functionalisation of the membrane with dopamine, the passage of the albumin solution at 20 mbars for 1 h does not cause the formation of a layer that clogs the pores, only of crystalline albumin deposits.

## Claims

1. A filtration system for blood or other biological fluids, whether or not they comprise cell suspensions, designed to treat fluid flow rates starting from microlitres/minute in a version for portable or domestic use, which comprises:
a) means for separating the plasma or suspending fluid from the corpuscular components;
b) a plasma or suspending fluid filtration unit designed to provide a fraction enriched in proteins, DNA, RNA, pathogens, inflammation mediators or other high-molecular-weight substances, and a fraction enriched in low-molecular-weight solutes;
c) a water recovery unit for further concentration of the fraction enriched in proteins, DNA, RNA, pathogens, inflammation mediators or other high-molecular-weight substances, and the integration thereof with the other units of the system via recycle streams and auxiliary streams to dilute the corpuscular components formed in a) and allow the operation of the filtration unit in step b);
d) means for detection of analytes of interest in the fraction enriched in proteins, DNA, RNA, pathogens, inflammation mediators or other high-molecular-weight substances from step c) and/or means for separating undesirable components present in said fraction;
e) means for recycling the fraction analysed or purified in step d), after mixing with the fraction enriched in low-molecular-weight solutes from step b) and with the corpuscular components from separation step a).

2. Blood filtration system according to claim 1 wherein the proteins of the enriched fraction are albumin and antibodies.

3. System according to claim 1 or 2 which also includes the addition of an auxiliary solution to water recovery unit c) and recycle of water from unit c) to units a) and b).

4. System according to claim 1, 2 or 3 wherein step d) comprises the removal of a portion of the enriched fraction to be subjected to analysis and recycling of the remaining portion, after mixing with the fraction enriched in low-molecular-weight solutes from step b) and with the corpuscular components from separation step a), for reconstitution of whole blood.

5. System according to claim 1, 2 or 3 wherein the separation means of step d) comprise an albumin-bound toxin adsorption unit and means for recycling the purified fraction, after mixing with the fraction enriched in low-molecular-weight solutes from step b) and with the corpuscular components from separation step a), for reconstitution of whole blood.

6. System according to any one of the preceding claims, wherein the water filtration and recovery units comprise a catecholamine-functionalised cellulose or polysulphone or polyethersulphone membrane.

7. System according to claim 6 wherein the catecholamine is dopamine.

8. System according to claim 6 or 7 wherein the membrane is inserted in a unit consisting of two compartments, in one of which fresh plasma or protein-enriched plasma flows, while a recycling stream consisting of plasma diluted in protein flows in the other.

9. System according to any one of claims 1 to 8 wherein the water recovery unit acts by direct or reverse osmosis.

10. A device configured to implement the system of claims 1 to 9.
